# EUROPEAN PATENT APPLICATION

(11) **EP 2 554 194 A1**
(43) Date of publication of application: **06.02.2013**
(21) Application number: 11762581.4
(22) Date of filing: 16.03.2011
(51) Int. Cl.: A61M 5/142, A61B 5/022, A61B 5/1455, A61M 5/00

(54) **INTEGRATED CIRCUIT AND MEDICAL DEVICE USING THE SAME**

(30) Priority: 31.03.2010 JP 2010080209
(71) Applicant: Terumo Kabushiki Kaisha, Tokyo 151-0072 (JP)
(72) Inventor: MORITA, Takashi, Ashigarakami-gun Kanagawa 259-0151 (JP); SUGAWARA, Yoshihisa, Ashigarakami-gun Kanagawa 259-0151 (JP); IWATA, Takeharu, Ashigarakami-gun Kanagawa 259-0151 (JP)
(74) Representative: TBK
(86) International application number: PCT/JP2011/056217
(87) International publication number: WO 2011/122343

(57) **Abstract**

To provide an integrated circuit which can be utilized as a common integrated circuit for a plurality types of medical devices including a sphygmomanometer, a blood glucose meter, a syringe pump and an infusion pump. It includes an amplification unit 20 which inputs a signal from an external sensor connected to outside of an integrated circuit 1 and an arithmetic control unit 31 which receives the signal processed by the amplification unit 10, and performs an arithmetic process for a control of devices or a calculation of a detection result according to an internal program. The external sensor connected to outside of the integrated circuit 1 and the internal program corresponding to the external sensor are changed, whereby the integrated circuit can be utilized as a common integrated circuit for the plurality types of different medical devices including the sphygmomanometer, the blood glucose meter, the syringe pump, and the infusion pump, according to a difference of external sensors.

## Description

### TECHNICAL FIELD

The present invention relates to an integrated circuit and medical devices such as a sphygmomanometer, a blood glucose meter, and a syringe pump and an infusion pump, which use the same.

### BACKGROUND ART

A measuring device requires dedicated processing circuits for each physical quantity of measurement or each type of sensors, and this requires a design and manufacture of the dedicated processing circuits according to various sensors, resulting in a high manufacturing cost.

Heretofore, in order to solve such a problem, a technique is proposed which seeks communalization and generalization of a processing circuit for a physical quantity sensor, and which provides a small-sized and cheap physical quantity sensor (patent document 1). According to the technique described in the patent document 1, a sensor head and a first electric processing circuit which converts the output of the sensor head into a common electric physical quantity are constituted on one substrate (a second substrate). Another substrate (a first substrate) for power supply is constituted, and a second electric processing circuit for processing unified electric physical quantity is constituted on still another substrate (a third substrate). As a result, according to the technique described in the patent document 1, it is possible to communalize the first and third substrates and to constitute various physical quantity sensors by replacing only the second substrate.

However, in the technique described in the patent document 1, the components (parts corresponding to the first and second substrates in the above-mentioned patent document 1) which receive the signal from a sensor and amplify the signal have to be constituted on separate substrates in accordance with the sensor heads, and communalization is not achieved. Moreover, in cases where there is a difference in the processing load such as light or heavy according to the types of sensor heads, if it attempts to completely communalize the substrates for processing the unified electric physical quantity, an expensive arithmetic processing circuit is required in order to accommodate the heaviest load, and this requires a communalization into an excessive high-performance substrate as the whole and results in high manufacturing cost on the contrary.

Therefore, heretofore, it is difficult to communalize integrated circuits for products in different categories which have a difference in the structures of the amplification units, each of which receives a signal from a sensor and performs an amplification process, and have different processing loads such as light and heavy. In particular, in the field of medical devices, it is just intended to make into one chip only the products in the limited categories as described in the patent document 2, and there is no integrated circuit which can be utilized as a common integrated circuit for a plurality of types of devices including a sphygmomanometer, a blood glucose meter, a syringe pump, and an infusion pump.

### RELATED ART LITERATURE

### PATENT DOCUMENT

Patent document 1: JP-B-3203788
Patent document 2: JP-A-2001-212098

### DISCLOSURE OF THE INVENTION

### PROBLEMS TO BE SOLVED BY THE INVENTION

An object of the present invention is to overcome the conventional approach and to provide an integrated circuit which can be utilized as a common integrated circuit for a plurality types of medical devices including sensing functions such as a sphygmomanometer, a blood glucose meter, a syringe pump and an infusion pump, whereby manufacturing cost is reduced.

Another object of the present invention is to provide a sphygmomanometer, a blood glucose meter, a syringe pump, and an infusion pump, which use the above-mentioned integrated circuit.

### MEANS TO SOLVE THE PROBLEMS

The above-described objects of the present invention are achieved by the following means.

An integrated circuit of the present invention includes: an amplification unit which inputs a signal from an external sensor connected to outside of the integrated circuit; and an arithmetic control unit which receives the signal processed by the amplification unit, and performs an arithmetic process for a control of medical devices or a calculation of a detection result according to an internal program, wherein the external sensor connected to outside of the integrated circuit and the internal program corresponding to the external sensor are changed, whereby the integrated circuit can be utilized as a common integrated circuit for a plurality types of medical devices including a sphygmomanometer, a blood glucose meter, a syringe pump, and an infusion pump, according to a difference of external sensors.

### EFFECT OF THE INVENTION

According to the present invention, there is provided an integrated circuit which can be utilized as a common integrated circuit for a plurality types of medical devices including a sphygmomanometer, a blood glucose meter, a syringe pump and an infusion pump, whereby manufacturing cost is reduced.

### BRIEF DESCRIPTION OF THE DRAWINGS

FIG. 1 is a block diagram schematically illustrating a structure of a platform integrated circuit of the embodiment of the present invention.
FIG. 2 is a drawing illustrating a manner of use of the platform integrated circuit in a sphygmomanometer according to the present embodiment.
FIG. 3 is a drawing illustrating a manner of use of the platform integrated circuit in a blood glucose meter according to the present embodiment.
FIG. 4 is a drawing illustrating a manner of use of the platform integrated circuit in a syringe pump according to the present embodiment.
FIG. 5 is a drawing illustrating a manner of use of the platform integrated circuit in an infusion pump according to the present embodiment.
FIG. 6 is a drawing illustrating use status for each device with respect to each component.
FIG. 7 is a drawing schematically illustrating a connection relationship to an input unit of the platform integrated circuit in the sphygmomanometer in FIG. 2.
FIG. 8 is a drawing schematically illustrating a connection relationship to an input unit of the platform integrated circuit in the blood glucose meter in FIG. 3.
FIG. 9 is a drawing schematically illustrating a connection relationship to an input unit of the platform integrated circuit in the syringe pump in FIG. 4.
FIG. 10 is a drawing schematically illustrating a connection relationship to an input unit of the platform integrated circuit in the infusion pump in FIG. 5.

### PREFERRED MODES FOR CARRYING OUT THE INVENTION

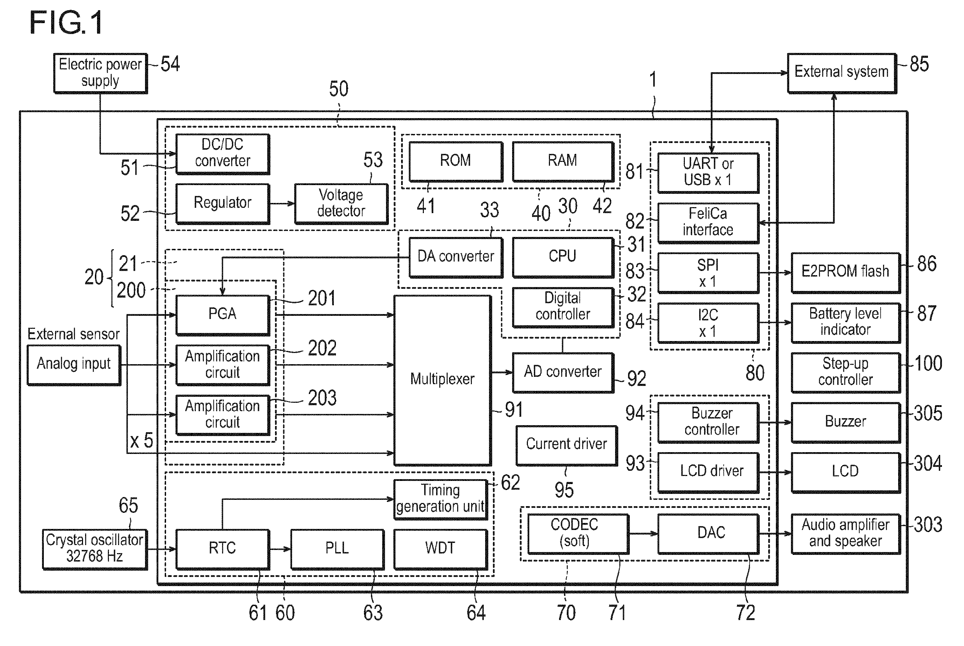

FIG. 1 is a block diagram schematically illustrating a structure of an integrated circuit of the embodiment of the present invention.

The platform integrated circuit 1 is an integrated circuit (IC) in which any of external sensors connected to outside of the integrated circuit and any of internal programs corresponding to the external sensor are changed, whereby the integrated circuit can be utilized as a common integrated circuit for a plurality types of medical devices including a sphygmomanometer, a blood glucose meter, a syringe pump, and an infusion pump, according to differences of the external sensors. The platform integrated circuit 1 is a system LSI which includes an analog circuit and a digital circuit on one chip.

### <Constitution of the integrated circuit>

The platform integrated circuit 1 includes an input unit 20 which receives an analog signal from outside, a control unit 30 including a CPU 31 which serves as an arithmetic control unit, and a memory unit 40.

The input unit 20 receives a plurality of inputs from outside and outputs a plurality of output signals. The input unit 20 includes an amplification unit 200 and an analog input unit 21 other than the amplification unit. The amplification unit 200 includes first through third amplification circuits 201, 202 and 203. The first amplification circuit 201 is a programmable gain amplifier (PGA) which is an amplification circuit of a differential gain adjustable type capable of adjusting a differential gain. The second and the third amplification circuits 202 and 203 are amplification circuits of fixed gain type of which gains are fixed. The amplification unit 200 is constituted so that recombination is possible to accommodate inputs from various external sensors, by changing connection relationship between a plurality of amplification circuits 201, 202 and 203 etc. This point is described later.

A selection unit 91 is provided between the input unit 20 and the control unit 30. The selection unit 91 is a multiplexer which subjects various kinds of signals from the amplification unit 200 and other analog input unit 21 to time division multiplexing, and transfers a plurality of signals to the CPU(arithmetic control unit) 31 of the control unit 30 with switching the signals at each predetermined time. An analog-to-digital converter (A/Dconverter) 92 is provided which subjects a signal output from the selection unit 91 to analog-to-digital conversion, and the signal from the selection unit 91 is digitized and is transferred to the CPU 31.

The memory unit 40 includes a ROM 41 and a RAM 42. The ROM 41 stores internal programs executed by the CPU 31. The RAM42 is used as temporary storage area for various kinds of data, and a working area for various calculations.

The control unit 30 includes the CPU 31 which serves as the arithmetic control unit, and a digital controller 32. The CPU 31 receives the signal from the amplification unit 200 and other analog input units 21, and performs a control of device, or a calculation of a detection result according to the internal program in the memory unit 40. The internal programs are stored according to the types of devices from among programs for the sphygmomanometer, for the blood glucose meter, for the syringe pump, and for the infusion pump. The digital controller 32 receives a command from the CPU 31, and controls each unit. The control unit 30 includes a digital-to-analog converter (DA converter) 33 for converting a digital signal output from the CPU 31 and so on into an analog signal in order to control the differential gain of the above-mentioned first amplification circuit 201 of differential gain adjustable type. The differential gain of the first amplification circuit 201 is set to a predetermined value by the signal converted into an analog value by the DA converter 33.

The platform integrated circuit 1 further includes a power supply unit 50, a clock unit 60, a voice generation unit 70, and an input-output unit 80, other than the above-described components.

The power supply unit 50 receives an electric power supply from an external power supply, converts it into suitable voltage, and supplies an electric power to each unit in the platform integrated circuit 1. The power supply unit 50 includes a DC-DC conversion unit 51, a regulator 52, and a voltage detector 53. The DC-DC conversion unit 51 steps up or steps down a voltage of the external power supply 54 so as to be a desired voltage level. The regulator 52 receives the voltage from the DC-DC conversion unit 51, and stabilizes it so as to be constant voltage. The voltage detector 53 monitors the voltage output from the regulator 52.

The clock unit 60 generates a clock signal and a timing signal which are necessary for operations of the CPU 31 serving as the arithmetic control unit and other components. The clock unit 60 includes, for example, an RTC (real-time clock) 61, a timing generation unit (timing generator) 62, and a PLL (Phase locked loop) 63.

The RTC 61 and the timing generation unit 62 include calendar functions, and can generate an interrupt signal at the designated time interval or the designated time on the basis of the oscillating frequency (for example, 32 kHz) of a crystal oscillator 65 connected to outside. The PLL 63 generates a high-speed clock for operating the CPU 31 from the frequency signal of the crystal oscillator 65. Furthermore, a WDT (Watch Dog Timer) 64 may be provided for monitoring the arithmetic control unit (microcomputer) to avoid a runaway.

The voice generation unit 70 is for outputting a voice from an external audio amplifier and a speaker 303, without using avoice IC which is an expensive component. Specifically, the voice generation unit 70 includes a voice decoder 71 constituted by software, and a digital-to-analog converter (DA converter) 72 for converting the digital data output from the voice decoder 71 into an analog signal. Unlike the present embodiment, the voice IC may be used as the voice generation unit 70.

The input-output unit 80 includes first through fourth interfaces 81-84, for example. The first interface 81 is for communicating with a personal computer and other external system, in order to take data into the personal computer and other external system 85. For example, as the first interface 81, an interface based on standards of UART (Universal Asynchronous Receiver Transmitter) or USB (Universal Serial Bus) is adoptable. The second interface 82 is an interface for communicating with a contactless IC card. For example, as the second interface 82, a FeliCa (registered trademark) interface is adoptable. The third interface 83 is an interface for storing data to an external nonvolatile memory 86, such as EEPROM (Electrically Erasable Programmable ROM) and a flash memory. The fourth interface 84 is an interface for communicating with an external battery level indicator (Fuel Gauge) 87. As the third and fourth interfaces 83 and 84, an SPI (serial peripheral interface), an I2C (Inter-Integrated Circuit) and so on are used, for example.

Furthermore, the platform integrated circuit 1 includes a liquid crystal display driver unit 93 which drives a liquid crystal panel (LCD) 304 in order to display a measurement result or the like on the LCD 304 connected to outside, a buzzer control unit (buzzer controller) 94 for sounding a warning sound (buzzer) from a buzzer 305 connected to outside, and a current driver unit 95 which is a constant current drive circuit for external sensors.

The platform integrated circuit 1 constituted as described above can be utilized as a common integrated circuit for a plurality types of medical devices including the sphygmomanometer, the blood glucose meter, the syringe pump, and the infusion pump, according to differences in the external sensors, by changing any of external sensors connected to an outside of the integrated circuit and any of internal programs corresponding to the external sensor. However, arithmetic controls of the syringe pump and the infusion pump are complicated in comparison with controls of the sphygmomanometer and the blood glucose meter. Therefore, in cases where the platform integrated circuit 1 is used as the integrated circuit for the syringe pump, or the integrated circuit for the infusion pump, the platform integrated circuit 1 can connect to a step-up controller 100 which serves as a functional complement integrated circuit complementing a part of functions of the CPU 31 (arithmetic control unit). The step-up controller 100 is a gate array or an FPGA (field programmable gate array). The step-up controller 100 is connectable with a terminal (not illustrated) of the platform integrated circuit 1, and is connected to the CPU 31 through the terminal and internal bus wiring (not illustrated) and the like. As a result, the step-up controller 100 receives a command from the CPU 31 and performs a part of arithmetic processes, and the CPU 31 and the step-up controller 100 can cooperate to perform the arithmetic control.

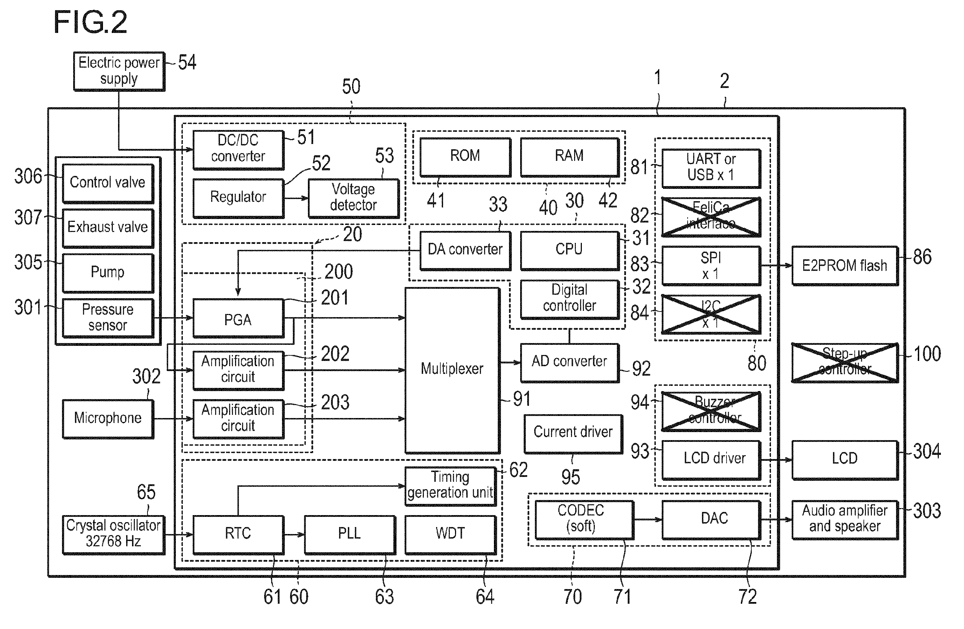

Respective elements provided in the platform integrated circuit 1 of the embodiment of the present invention described above, are classified roughly into a first element group, which is used in common regarding all of the plurality types of medical devices including the sphygmomanometer, the blood glucose meter, the syringe pump and the infusion pump, and a second element group, which is used only regarding a part of the plurality types of medical devices. This point will be described with reference to FIG. 2 through FIG. 6.

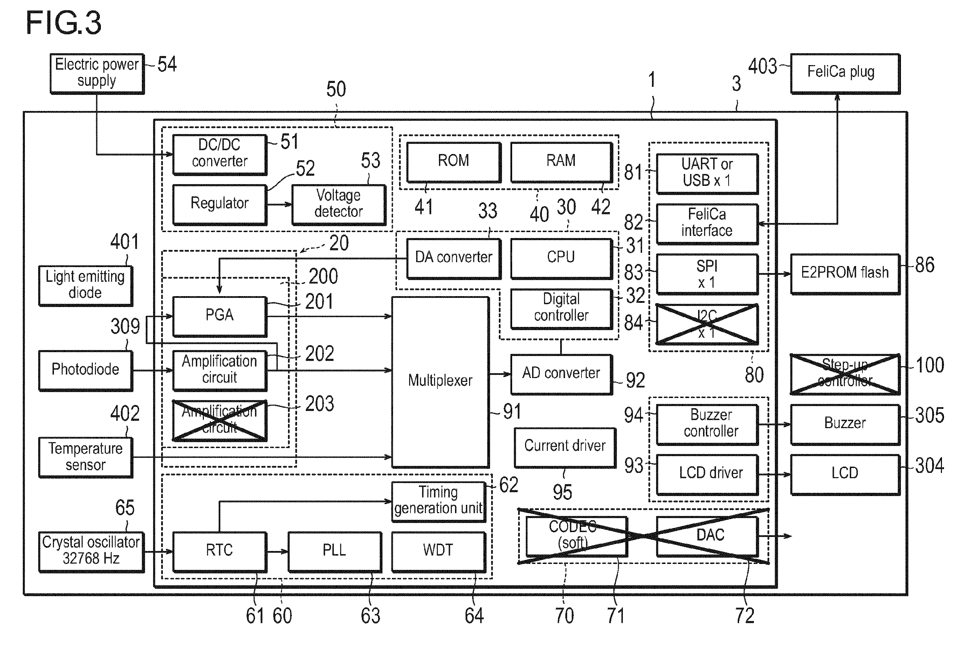

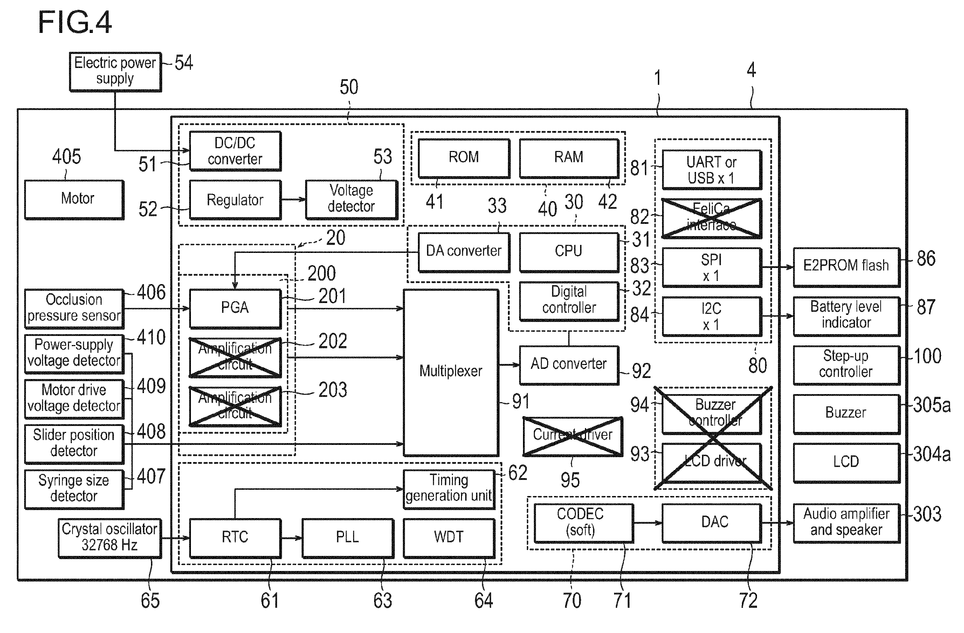

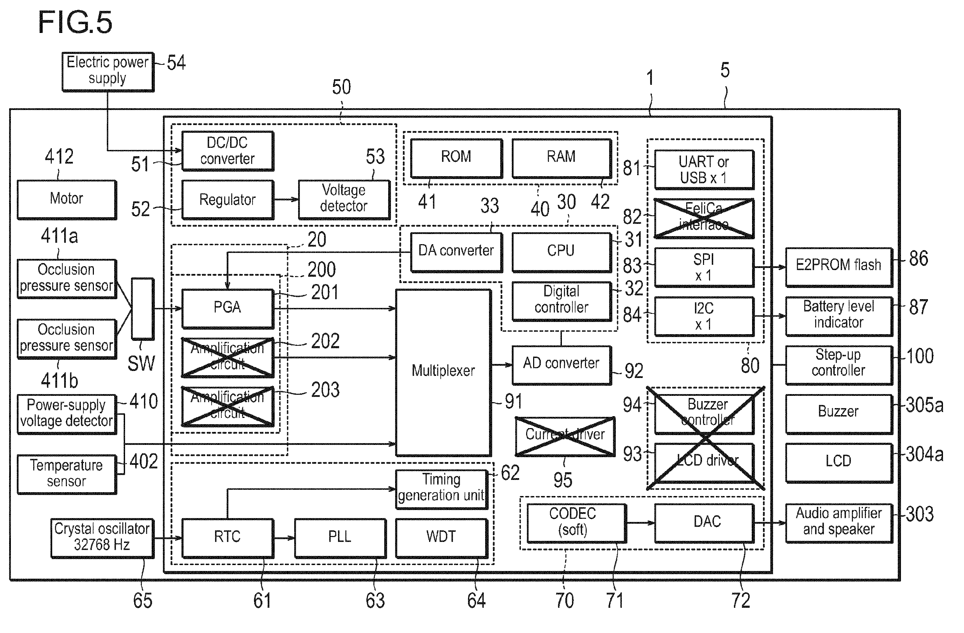

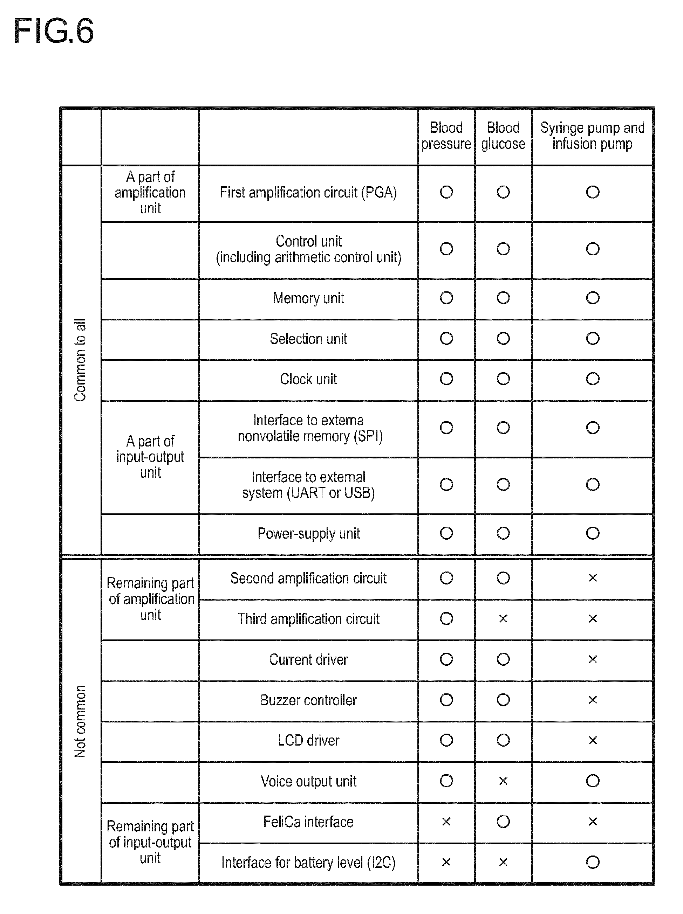

FIG. 2 is a drawing illustrating a manner of use of the platform integrated circuit in the sphygmomanometer according to the present embodiment. FIG. 3 is a drawing illustrating a manner of use of the platform integrated circuit in the blood glucose meter according to the present embodiment. FIG. 4 is a drawing illustrating a manner of use of the platform integrated circuit in the syringe pump according to the present embodiment. FIG. 5 is a drawing illustrating a manner of use of the platform integrated circuit in the infusion pump according to the present embodiment. In FIG. 2 through FIG. 5, mark x is presented on each element which is not used by each of devices. FIG. 6 is a drawing illustrating the use status for each device with respect to each component.

As illustrated in FIG. 6, components included in the first element group which is used in common regarding all devices include following components. Specifically, the first element group includes: (a) some amplification circuits included in the amplification unit 200, specifically, the first amplification circuit 201 which is a programmable gain amplifier (PGA); (b) the control unit 30 (including the CPU 31 which serves as the arithmetic control unit) ; (c) the memory unit 40; (d) the selection unit 91; (e) the clock unit 60; and (f) some interfaces included in the input-output unit 80, specifically, the first interface 81 for the external system 85 and the third interface 83 for the external nonvolatile memory 86.

On the other hand, components of the second element group include the following. Specifically, the second element group includes: (g) remaining amplification circuits that are not included in the above-described first element group among the amplification circuits included in the amplification unit 200, specifically, the second amplification circuit 202 and the third amplification circuit 203; (h) the current driver unit 95 which provides current to the external sensor in cases where the platform integrated circuit 1 is used as the integrated circuit for the sphygmomanometer, or the integrated circuit for the blood glucose meter; (i) the internal embedded buzzer control unit 94 and the internal embedded liquid crystal display driver unit (LCD driver unit) 93 which are utilized in cases where the platform integrated circuit 1 is used as the integrated circuit for the sphygmomanometer, or the integrated circuit for the blood glucose meters; (j) the voice generation unit 70 for outputting the voice, in cases where the platform integrated circuit 1 is used as the integrated circuit for the sphygmomanometer, the integrated circuit for the syringe pump, or the integrated circuit for the infusion pump; and (k) the remaining interfaces that are not included in the above-described first element group among the interfaces included in the input-output unit 80, specifically the second interface 82 for the contactless IC card, and the fourth interface 84 for the battery level indicator.

In this way, the platform integrated circuit 1 of the present embodiment can be constituted, in which not only first element group used in common regarding all devices (that is, components as taking the greatest common measure) is made into one chip, but also the second element group is included on the chip.

Although an LCD and a buzzer (alarm) are provided in the syringe pump 4 or the infusion pump 5 as illustrated in FIG. 4 and FIG. 5, it is not necessary to use the buzzer control unit 94 and the LCD driver unit 93 embedded in the platform integrated circuit 1 for these LCD 304a and buzzer 305a, and a high-performance buzzer control unit and an LCD driver unit (not illustrated) connected on outside may be used. On the other hand, for the LCD 304 in the sphygmomanometer and the blood glucose meter, and the buzzer 305 in the sphygmomanometer, the buzzer control unit 94 and the LCD driver unit 93 embedded in the platform integrated circuit 1 are used.

In this way, in accordance with the types of devices (that is, in accordance with the internal programs), such as the sphygmomanometer, the blood glucose meter, the syringe pump and the infusion pump, it is set whether using the internal embedded component (s) provided, or using external connected component which has same type of function as the internal embedded component and has different performance therefrom, as at least one component (in this embodiment, two of the buzzer control unit 94 and the liquid crystal display driver unit 93) included in the second element group.

Components provided in the platform integrated circuit 1 as components to be used in common regarding some apparatuses of the plurality types of devices such as the sphygmomanometer, the blood glucose meter, the syringe pump, and the infusion pump may be used as elements which have different roles in accordance with the devices. Specifically, as illustrated in FIG. 6, the first amplification circuit (PGA) 201 is used in common to the sphygmomanometer, the blood glucose meter, the syringe pump and the infusion pump. However, in cases where the platform integrated circuit 1 is used as the integrated circuit for the sphygmomanometer, the integrated circuit for the syringe pump, or the integrated circuit for the infusion pump, the first amplification circuit 201 functions as an amplification circuit of a first stage which receives the signal from the external sensor, whereas in cases where the platform integrated circuit 1 is used as the integrated circuit for the blood glucose meter, the first amplification circuit 201 functions as an amplification circuit of the following stage.

The platform IC constituted as described above can be used as the integrated circuit for the sphygmomanometer, the integrated circuit for the blood glucose meter, the integrated circuit for the syringe pump, and the integrated circuit for the infusion pump, as described later. Hereinafter, it will be described a manner of use of the platform integrated circuit in each devices and operation of the platform IC of the present embodiment.

### <a case utilized as the integrated circuit for the sphygmomanometer>

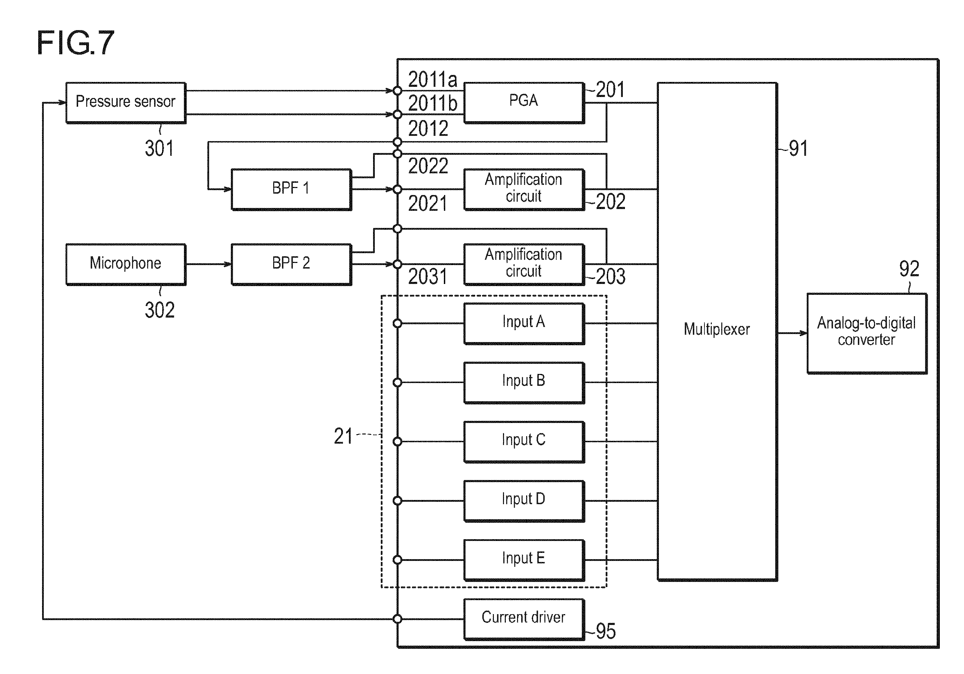

FIG. 2 described above is a drawing illustrating a manner of use of the platform integrated circuit in a sphygmomanometer according to the present embodiment. The sphygmomanometer 2 is a medical device for measuring human blood pressure. FIG. 7 is a drawing schematically illustrating a connection relationship to an input unit of the platform integrated circuit in the sphygmomanometer.

With reference to FIG. 2, in cases where the platform integrated circuit 1 is used as the integrated circuit for the sphygmomanometer, the integrated circuit 1 is provided with, as external electric components, a pressure sensor 301, a microphone unit 302, audio amplifier and speaker 303, an LCD 304, a pump 305, a control valve 306, an exhaust valve 307, a nonvolatile memory 86, and a crystal oscillator 65 and so on.

As illustrated in FIG. 7, in a plurality of amplification circuits (amplifiers) in the embodiment of the present invention, the first amplification circuit (PGA) 201 is constituted so as to amplify the difference between the signals input into first input terminal units 2011a and 2011b and output amplified signals to a first output terminal unit 2012. The second amplification circuit 202 is constituted so as to amplify the signal input into a second input terminal unit 2021 and output amplified signal to a second output terminal unit2022. The third amplification circuit 203 is constituted so as to amplify the signal input into the third input terminal 2031. In this way, each of the amplification circuits 201 to 203 amplifies the signal input into each of the input terminal units which differ from each other, and outputs amplified signal to each of the output terminal units which differ from each other. The first input terminal units 2011a and 2011b, the first output terminal unit 2012, the second input terminal unit 2021, the second output terminal unit 2022, and the third input terminal 2031 are exposed outside, so that the integrated circuit can accommodate each device by changing a connection state to an outside.

On the premise of the above structures, it will be described a case of utilizing the platform integrated circuit 1 as the integrated circuit for the sphygmomanometer. In this case, the pressure sensor 301, which detects the pressure of the arm cuff unit through which a subject's upper arm is inserted, is connected to the first input terminal units 2011a and 2011b of the first amplification circuit (PGA) 201 of a differential gain adjustable type, and the first output terminal unit 2012 and the second input terminal unit 2021 of the second amplification circuit are connected through a bandpass filter BPF1 which is a first external circuit.

Moreover, an audio signal (detection signal of voltage) is input through a bandpass filter BPF2 from the microphone unit 302 provided at the arm cuff unit into the third amplification circuit 203 and is amplified thereby. The control valve 306, the exhaust valve 307, and the pump 305 are connected to the platform integrated circuit 1 so that each part can be driven in response to an instruction from the control unit 30.

In a state where the above-described connection is made, the pressure signal (detection signal) detected by the pressure sensor 301 provided at the arm cuff unit is input into the first amplification circuit (PGA) 201 as an analog differential voltage signal, is applied a differential amplification by the first amplification circuit 201, and after that, is input into the selection unit 91 which is a multiplexer as a signal for one channel. Since the pressure sensor 301 has a variation depend on sensors, it is preferable to set the gain of the first amplification circuit 201 as an amplifier of a first stage to be an appropriate value by the DA converter 33.

Furthermore, the pressure signal subjected to the differential amplification by the first amplification circuit 201 is amplified and filtered by the bandpass filter BPF1 and the second amplification circuit 202, and after that, is input into the selection unit 91 as a signal for one channel. By filtering the pressure signal by the bandpass filter BPF1, it is possible to extract only pulse wave component which can be directly utilized for blood pressure measurement from the pressure signal. The pulse wave component can be utilized for oscillometric blood pressure measurement.

On the other hand, the audio signal (detection signal of voltage) from the microphone unit 302 is amplified and filtered by the bandpass filter BPF2 and the third amplification circuit 203, and after that, is input into the selection unit 91 as a signal for one channel. The audio signal from the microphone can be utilized for the blood pressure measurement by a Korotkov's sound method.

Each of signals of amplified pressure signal, the pulse wave component, and the sound is subjected to a time division multiplexing by the selection unit 91 which is a multiplexer, and is subjected to a digital conversion by the AD converter to provide the CPU 31.

The CPU 31 instructs the pump 305 to provide air to the arm cuff unit at the time of starting the blood pressure measurement, in accordance with a sphygmomanometer program as an internal program. Moreover, the CPU 31 can store each of signals of the pressure signal which is subjected to the digital conversion, the pulse component, and the audio signal in the nonvolatile memory 86 through the interface 83.

When the CPU 31 determines that the arm cuff unit has been pressurized to predetermined pressure based on the pressure signal which is subjected to the differential amplification by the first amplification circuit (PGA) 201, the CPU 31 controls a drive of the control valve 306 based on the pressure signal so that the air in the arm cuff unit is decompressed at a fixed speed. When applying the oscillometric blood pressure measurement, blood-pressure values (highest blood pressure and lowest blood pressure) are calculated by detecting a point where the pulse component rapidly increases and a point where the pulse component rapidly decreases based on the signal of the pulse component and the pressure signal, and the measurement result is displayed on the LCD 304. When applying the Korotkov's sound method, the blood-pressure values (highest blood pressure and lowest bloodpressure) are calculated by detecting an occurring point of Korotkov's (K) sound and a disappearing point thereof based on the audio signal and the pressure signal. If an exsufflation is required, the CPU 31 controls the exhaust valve 307 to drive.

### <a case utilized as the integrated circuit for the blood glucose meter>

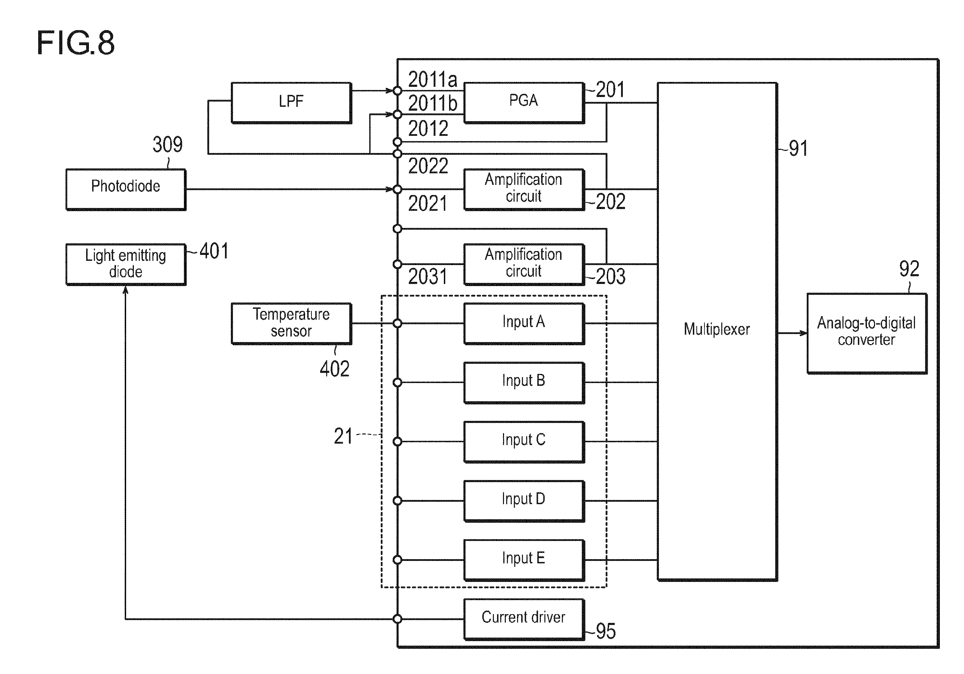

FIG. 3 is a drawing illustrating a manner of use of the platform integrated circuit in a blood glucose meter 3 according to the present embodiment. The blood glucose meter 3 is for measuring optically a degree of coloration of a (not illustrated) specimen (for example, a paper in which reagents such as glucose oxidase are impregnated) which is colored in accordance with the amount of glucose in blood. FIG. 8 is a drawing schematically illustrating a connection relationship to an input unit of the platform integrated circuit in the blood glucose meter.

As illustrated in FIG. 3, in cases where the platform integrated circuit 1 is used as the integrated circuit for the blood glucose meter, the integrated circuit 1 is provided with, as external electric components, a photodiode 309, a light emitting diode 401, a temperature sensor 402 such as a thermistor, the LCD 304, the nonvolatile memory 86, a plug 403 for a contactless IC card, the buzzer 305, and the crystal oscillator 65.

As illustrated in FIG. 8, in cases where the platform integrated circuit 1 is utilized as the integrated circuit for the blood glucose meter, the photodiode 309 for measuring optically a degree of coloration of an examination part which is colored in accordance with the amount of glucose in blood is connected to the second input terminal unit 2021 of the second amplification circuit 202 of a fixed gain type, and the second output terminal unit 2022 and one of the first input terminal units 2011a of the first amplification circuit (PGA) 201 are connected through a low pass filter LPF as a second external circuit. Furthermore, the second output terminal unit 2022 is connected to another of the first input terminal units 2011b of the first amplification circuit (PGA) 201 without passing through the low pass filter LPF. The temperature sensor 402 such as the thermistor is connected to the input A which is the input unit. The light emitting diode 401 is connected to the current driver unit 95 through a terminal which is not illustrated.

In a state where the above-described connection is made, current is provided to the light emitting diode 401 through the current driver unit 95 under a control by the CPU 31 which executes the blood glucose meter program as the internal program. In this way, the light emitting diode 401 irradiates light to the specimen in which a subj ect' s blood is transfused. At that time, the light signal (detection signal of current) detected by the photodiode 309 included in the blood glucose meter is subjected to a current / voltage conversion and an amplification by the second amplification circuit 202, which constitutes a transimpedance amplifier with an external resistor which is not illustrated, and after that, is input into the selection unit 91 as one channel.

Furthermore, the light signal amplified by the second amplification circuit 202 is input into the first amplification circuit (PGA) 201 as one of the differential signals, and is input into the first amplification circuit (PGA) 2011 through the low pass filter LPF as another of the differential signals.

The first amplification circuit 201 amplifies a difference between the light signal input through the low pass filter LPF and the light signal without passing through the low pass filter LPF. Accordingly, it is possible to extract the high-frequency component utilized for measurement of the blood glucose level in the light signals with high precision. The signal amplified by the first amplification circuit (PGA) 201 is input into the selection unit 91 as one channel. The gain of the first amplification circuit (PGA) 201 is set to an appropriate value by the DA converter 33.

In accordance with the blood glucose meter program as the internal program, the CPU 31 detects the degree of coloration based on the signal amplified by the first amplification circuit (PGA) 201, and performs an arithmetic control to calculate a blood glucose level therefrom. At that time, the influence of temperature can be compensated based on the signal from the temperature sensor 402.

### <a case utilized as the integrated circuit for the syringe pump>

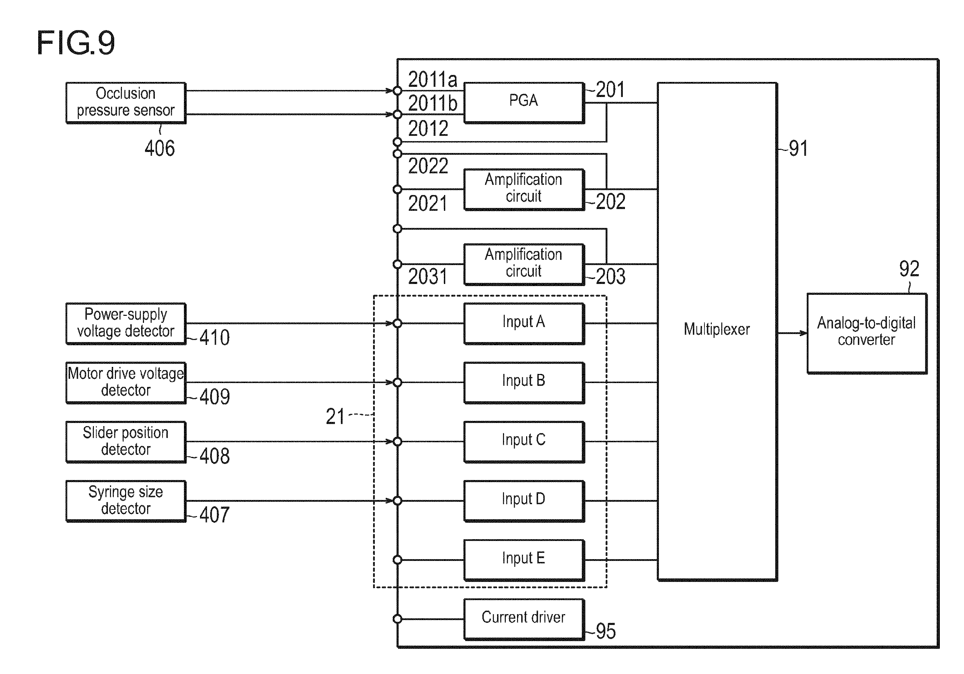

FIG. 4 is a drawing illustrating a manner of use of the platform integrated circuit in the syringe pump 4 according to the present embodiment. The syringe pump 4 is an apparatus which sets a syringe, moves a plunger of syringe by slider means driven by a motor, whereby the contents in the syringe is precisely injected into the inside of the body. The syringe pump is a medical device which has a main purpose to perform nutrition and blood transfusion to a patient, and to inject medical fluid such as a chemotherapeutic drug and an anesthetic drug in high accuracy for relatively long time in an intensive care unit (ICU) etc. and the syringe pump has a superior function of medical fluid flow control in comparison with other types of pumps. FIG. 9 is a drawing schematically illustrating a connection relationship to an input unit of the platform integrated circuit in the syringe pump.

As illustrated in FIG. 4, in cases where the platform integrated circuit 1 is used as the integrated circuit for the syringe pump, the integrated circuit 1 is provided with, as external electric components, a motor 405 used as a source of driving the slider means to move the plunger of syringe, an occlusion pressure sensor 406 which detects the occlusion pressure for monitoring the occlusion state of the syringe, a syringe size detector 407 which detects a diameter size of the syringe, a slider position detector 408 for detecting a position of the slider means which is moved, a motor drive voltage detector 409, a power-supply voltage detector 410, audio amplifier and speaker 303, the LCD 304a, the buzzer 305a, the nonvolatile memory 86, and the crystal oscillator 65 and so on. Moreover, the step-up controller 100 for complementing the arithmetic control function of the CPU 31 is connected to the platform integrated circuit 1.

As illustrated in FIG. 9, the occlusion pressure sensor 406 is connected to the first input terminal units 2011a and 2011b of the first amplification circuit (PGA) 201 of a differential gain adjustable type. The syringe size detector 407, the slider position detector 408, the motor drive voltage detector 409, and the power-supply voltage detector 410 are connected so that corresponding signals are input into the inputs A to D which are the analog input units 21 of the platform integrated circuit 1, respectively. The motor 405 is connected so that the instruction from the CPU 31 and the step-up controller 100 can be received.

In a state where the above-described connection is made, the occlusion pressure detected by the occlusion pressure sensor 406 included in the syringe pump is input into the first amplification circuit 201 as a differential voltage signal (detection signal) to be amplified, and is input into the selection unit 91 as a signal for one channel. The gain of the first amplification circuit 201 is set to an appropriate value by the DA converter 33.

The amplified occlusion pressure and each of signals of power supply voltage, motor drive voltage, slider position detection, and syringe size detection are subjected to a time division multiplexing in the selection unit 91 which is a multiplexer, and are subjected to a digital conversion by the AD converter. Each signal which is subjected to the digital conversion is stored in the external nonvolatile memory 86 through the third interface 83 under a control of the CPU 31, and can be utilized as a signal for control of the syringe pump, and a failure detection signal.

Specifically, the CPU 31 and the step-up controller 100 calculate the syringe size from the syringe size detection signal in accordance with a program for the syringe pump as the internal program. Then, the plunger of syringe is moved by a feedback control or the like based on the detection result of the slider position, so that a flow amount and an integrated amount become the amounts which the user inputs beforehand for setting. Considering an influence of a change of the occlusion pressure and so on to the feedback control at the time of starting the drive, the CPU 31 and the step-up controller 100, which receives the occlusion pressure signal from the occlusion pressure sensor 406, can also perform a motor control calibration by compensating parameters for the feedback control. Moreover, the occlusion pressure is also used in order to output the failure detection signal. An actual f low amount, the integrated amount and so on are detected based on the current slider position. Various detection results such as the flow amount, the integrated amount, and the occlusion pressure are displayed on the LCD 304a, and it is alarmed by the buzzer 305a if necessary. The functional assignment with the CPU 31 and the step-up controller 100 can be suitably set by the internal program.

Moreover, for the LCD 304a of the syringe pump and the buzzer 305a, it is prefer to set by the internal program so as to use a high-performance buzzer control unit and an LCD driver unit (not illustrated) connected on outside, instead of using the buzzer control unit 94 and the LCD driver unit 93 embedded in the platform integrated circuit 1. This point is same in the case of the infusion pump described later.

### <a case utilized as the integrated circuit for the infusion pump>

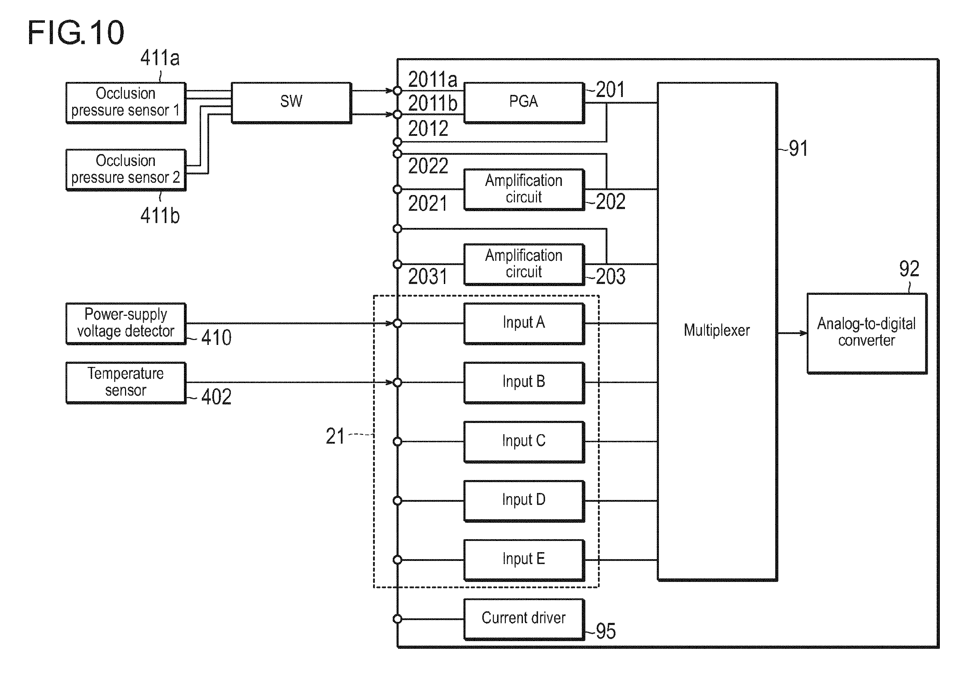

FIG. 5 is a drawing illustrating a manner of use of the platform integrated circuit in the infusion pump 5 according to the present embodiment. The infusion pump 5 is an apparatus which performs a liquid supply by peristalsis realized by pressing the periphery surface of the infusion tube to the downstream side one by one by a plurality of fingers provided along a longitudinal direction of the tube. The plurality of fingers is driven by a motor through a drive mechanism. Such infusion pump is a medical device for dosing a drug for the patient continuously with a predetermined dosage for intravenous drip per hour, and for example, it is used to give an intravenous drip injection correctly to the patient after an operation. FIG. 10 is a drawing schematically illustrating a connection relationship to an input unit of the platform integrated circuit in the infusion pump.

As illustrated in FIG. 5, in cases where the platform integrated circuit 1 is used as the integrated circuit for the infusion pump, the integrated circuit 1 is provided with, as external electric components, two occlusion pressure sensors 411a and 411b which detect the occlusion pressure in order to monitor the occlusion state of the infusion tube in a plurality of positions, the motor 412 which drives a drive mechanism for driving the plurality of fingers, the temperature sensors 402 such as the thermistor, the power-supply voltage detector 410, the audio amplifier and speaker 303, the LCD 304a, the buzzer 305a, the nonvolatile memory 86, and the crystal oscillator 65. Moreover, the step-up controller 100 for complementing the arithmetic control function of the CPU 31 is connected to the platform integrated circuit 1.

As illustrated in FIG. 10, the two occlusion pressure sensors 411a and 411b included in the infusion pump are connected to the first input terminal units 2011a and 2011b of the first amplification circuit (PGA) 201 through the switch SW as a differential voltage signal (detection signal). The temperature sensor 402 and the power-supply voltage detector 410 are connected so that each signal is input into the inputs A and B which are the analog input units 21 of the platform integrated circuit 1, respectively. The motor 412 is connected so that the instruction from the CPU 31 and the step-up controller 100 can be received.

In a state where the above-described connection is made, any one occlusion pressure, selected by the switch SW which switches in time or the like, among the occlusion pressure 1 and the occlusion pressure 2 detected by the two occlusion pressure sensors 411a and 411b included in the infusion pump, is input into the first amplification circuit 201 as the differential voltage signal (detection signal), amplified by the first amplification circuit 201, and after that, input into the selection unit 91 which is a multiplexer as a signal for one channel. The gain of the first amplification circuit 201 is set to an appropriate value by the DA converter 33.

The amplified occlusion pressure 1 and 2 and each signal of power supply voltage and temperature are subjected to a time division multiplexing in the selection unit 91 which is a multiplexer, and are subjected to a digital conversion by the AD converter. Each signal which is subjected to the digital conversion is stored in the external nonvolatile memory 86 through the third interface 83 under a control of the CPU 31, and can be utilized as a signal for control of the infusion pump, and a failure detection signal.

Specifically, the CPU 31 and the step-up controller 100 generate a motor rotation signal so that an infusion is made continuously for a predetermined time (or to be the integrated amount which is predetermined) at a flow per time which is set beforehand by the user, cause the motor to rotate by the rotation signal, and press the external surface of the infusion tube by the fingers according to a rotation of the motor, whereby the flow amount of the infusion is controlled. Based on the occlusion pressure 1 and the occlusion pressure 2 obtained from the two occlusion pressure sensors, the occlusion on upper stream side and downstream side of the infusion tube are detected, for example, and an alarm display or the like is made according to the pressure when the occlusion is detected.

Considering that a hardness of the external surface of the infusion tube changes according to temperature conditions, the CPU 31 and the step-up controller 100, which receives the detected temperature from the temperature sensor, can also perform the motor control calibration.

As explained above, the platform integrated circuit of the embodiment of the present invention can have following effects.
(a) According to the platform integrated circuit of the present embodiment, since it can be utilized as a common integrated circuit among a plurality kinds of different medical devices including the sphygmomanometer, the blood glucose meter, the syringe pump and the infusion pump which include a sensing function, it is possible to communalize the components of the plurality kinds of devices, and therefore, it is possible to realize a further cost reduction of the components for devices and the devices using the components.
(b) According to the platform integrated circuit of the present embodiment, it is possible to make into one chip not only the first element group used in common for all devices of the plurality kinds of different medical devices (components as taking the greatest common measure), but also the second element group used only for some devices. Therefore, since the range of the elements to be implemented by making into one chip is expanded and the number of external components decreases, the size of the overall circuit can be reduced as well as further cost reduction for devices can be realizable.
(c) According to the platform integrated circuit of the present embodiment, the internal program sets, in accordance with the plurality kinds of medical devices, whether using an internal embedded component or using an external connection component which has the same function as the internal embedded component and has different performance therefrom. Therefore, in cases where different performances of the function are required in accordance with the application of devices, it is not necessary to provide an expensive internal embedded component to accommodate the highest functional performance.
(d) According to the platform integrated circuit of the present embodiment, the platform integrated circuit includes a plurality of amplification circuits that amplifies the signals input into the input terminal units which are different to each other and outputs the amplified signals to the output terminal units which are different to each other, and a connection status between the input terminal units and the output terminal units is changed in accordance with the type of external sensor selectively connected to the integrated circuit, whereby, among the plurality of amplification circuits, an order can be changed between the amplification circuit of the first stage which receives the signals from the external sensor first, and the amplification circuit of the following stage. Therefore, it is also possible to communalize the input units which are required to different functions according to types of sensor heads.
(e) According to the platform integrated circuit of the present embodiment, some components such as the first amplification circuit 201 can be used as elements to serve different functions according to the devices, even if the components are included in the platform integrated circuit 1 to be used in common regarding some devices among the plurality kinds of different medical devices such as a sphygmomanometer, a blood glucose meter, a syringe pump, and an infusion pump. Therefore, it is possible to reduce necessity for providing separate circuit elements for each different function.
(f) According to the platform integrated circuit of the present embodiment, the platform integrated circuit can be connected to a functional complement integrated circuit which complements a part of functions of an arithmetic control unit in accordance with the type of the plurality kinds of medical devices. Therefore, in cases where there is a difference in the processing load such as light or heavy according to the types of sensor heads, it is not necessary to provide an expensive calculation process circuit to accommodate the heaviest load, and it is possible to avoid a communalization into an excessive high-performance substrate as the whole.

The integrated circuit and the sphygmomanometer, the blood glucose meter, the syringe pump, and the infusion pump which use the integrated circuit according to the embodiments of the present invention has been explained, but the present invention is not limited to the above-described embodiments. Amodification, an addition and an omission can be made suitably within a scope of invention without departing from the technical idea of the claims.

This application is based on the Japanese patent application No. 2010-080209, filed on March 31, 2010, and the entire contents of which are incorporated herein by reference.

### Explanation of reference numeral

- 1: platform integrated circuit,
- 2: sphygmomanometer,
- 3: blood glucose meter,
- 4: syringe pump,
- 5: infusion pump,
- 20: input unit,
- 30: control unit,
- 31: CPU (arithmetic control unit),
- 40: memory unit,
- 50: power supply unit,
- 60: clock unit,
- 70: voice generation unit,
- 80: input-output unit,
- 91: selection unit,
- 95: current driver unit,
- 93: LCD driver,
- 94: buzzer control unit,
- 100: step-up controller (functional complement integrated circuit),
- 200: amplification unit,
- 201: first amplification circuit (PGA),
- 202: second amplification circuit,
- 203: third amplification circuit,
- 301: pressure sensor (external sensor),
- 302: microphone unit (external sensor),
- 306: photodiode (external sensor),
- 406, 411a, b: occlusion pressure sensor (external sensor),
- 2011a, b: first input terminal,
- 2012: first output terminal,
- 2021: second input terminal,
- 2022: second output terminal, and
- 2031: third input terminal.

## Claims

1. An integrated circuit comprising:
an amplification unit which inputs a signal from an external sensor connected to outside of the integrated circuit; and
an arithmetic control unit which receives the signal processed by the amplification unit, and performs an arithmetic process for a control of medical devices or a calculation of a detection result according to an internal program,
wherein the external sensor connected to outside of the integrated circuit and the internal program corresponding to the external sensor are changed, whereby the integrated circuit can be utilized as a common integrated circuit for a plurality types of different medical devices including a sphygmomanometer, a blood glucose meter, a syringe pump, and an infusion pump, according to a difference of external sensors.

2. The integrated circuit as claimed in claim 1, wherein the integrated circuit interiorly includes:
a first element group used in common regarding all of the plurality types of different medical devices; and
a second element group used only regarding a part of the plurality types of different medical devices.

3. The integrated circuit as claimed in claim 2, wherein
in accordance with a type among the plurality types of medical devices, the internal program sets whether to use an internal embedded component provided as at least one component in the second element group, or using an external connected component which has the same kind of function as that of the internal embedded component and has a different performance from that of the internal embedded component.

4. The integrated circuit as claimed in claim 2 or 3, wherein the first element group includes:
(a) at least a part of amplification circuits included in the amplification unit;
(b) the arithmetic control unit;
(c) a memory unit which stores the internal program;
(d) a selection unit which is provided between the amplification unit and the arithmetic control unit, and transfers various kinds of signals from the amplification unit and other analog input to the arithmetic control unit while switching the various kinds of signals with a predetermined cycle;
(e) a clock unit which generates a clock signal and a timing signal for the arithmetic control unit;
(f) at least a part of interfaces included in an input-output unit for communicating with outside; and
(g) a power supply unit which receives electric power supply from outside and provides the electric power to each part of the integrated circuit.

5. The integrated circuit as claimed in claim 4, wherein the second element group includes:
(h) remaining amplification circuits which are not included in the first element, in the amplification units;
(i) a current driver unit which provides current to the external sensor in cases where the integrated circuit is used as the integrated circuit for the sphygmomanometer, or the integrated circuit for the blood glucose meter;
(j) an internal embedded buzzer control unit and an internal embedded liquid crystal display driver unit which are utilized in cases where the integrated circuit is used as the integrated circuit for the sphygmomanometer, or the integrated circuit for the blood glucose meter;
(k) a voice generation unit for outputting a voice, in cases where the integrated circuit is used as the integrated circuit for the sphygmomanometer, the integrated circuit for the syringe pump, or the integrated circuit for the infusion pump; and
(l) remaining interfaces which are not included in the first element group, in the input-output units for communicating with outside.

6. The integrated circuit as claimed in any one of claims 1 to 5, wherein
the amplification unit includes a plurality of amplification circuits which amplify the signals input into different input terminal units and output amplified signals to different output terminal units, and
connections between the input terminal units and the output terminal units are changed in accordance with the type of the external sensor selectively connected to the integrated circuit, and thus an order of the amplification circuit of a first stage which receives the signal from the external sensor first and the amplification circuits of following stages can be switched among the plurality of the amplification circuits.

7. The integrated circuit as claimed in claim 6, wherein the amplification unit includes:
a first amplification circuit of a differential gain adjustable type, which processes the signal input into a first input terminal unit and outputs the signal to a first output terminal unit;
a second amplification circuit of a fixed gain type, which processes the signal input into a second input terminal unit and outputs the signal to a second output terminal unit; and
a third amplification circuit of a fixed gain type, which processes the signal input into a third input terminal unit and outputs the signal, wherein
in cases where the integrated circuit is utilized as the integrated circuit for the sphygmomanometer, a pressure sensor detecting a pressure of an arm cuff unit, into which a subject's upper arm is inserted, is connected to the first input terminal unit of the first amplification circuit of the differential gain adjustable type, the first output terminal unit and the second input terminal unit are connected through a first external circuit whereas a microphone unit detecting a sound of blood-flow in the upper arm is connected to the third input terminal unit, and output signals generated by the first amplification circuit, the second amplification circuit and the third amplification circuit respectively are subjected to analog-to-digital conversion to be provided to the arithmetic control unit,
in cases where the integrated circuit is utilized as the integrated circuit for the blood glucose meter, an optical sensor for measuring optically a degree of coloration of examination part which is colored in accordance with the amount of glucose in blood is connected to the second input terminal unit of the second amplification circuit of the fixed gain type, the second output terminal unit and the first input terminal unit are connected through the second external circuit, and output signals generated by the first amplification circuit and the second amplification circuit respectively are subjected to the analog-to-digital conversion to be provided to the arithmetic control unit, and
in cases where the integrated circuit is utilized as the integrated circuit for the syringe pump, or the integrated circuit for the infusion pump, an occlusion pressure sensor monitoring an occlusion state of a syringe or an infusion tube is connected to the first input terminal unit of the first amplification circuit of the differential gain adjustable type, and an output signal generated by the first amplification circuit is subjected to the analog-to-digital conversion to be provided to the arithmetic control unit.

8. The integrated circuit as claimed in any one of claims 1 to 7, wherein the integrated circuit is capable of connecting to a functional complement integrated circuit which complements a part of functions of the arithmetic control unit according to a type among the plurality types of medical devices.

9. The integrated circuit as claimed in claim 8, wherein
the integrated circuit is a system LSI in which an analog circuit and a digital circuit are mixed, and
the functional complement integrated circuit is a gate array or a field programmable gate array.

10. The integrated circuit as claimed in claim 8 or 9, wherein
the functional complement integrated circuit complements arithmetic functions of the integrated circuit by a parallel processing with the arithmetic control unit.

11. The integrated circuit as claimed in any one of claims 8 to 10, wherein
in cases where the integrated circuit is used as the integrated circuit for the syringe pump or the integrated circuit for the infusion pump, the functional complement integrated circuit is connected.

12. A sphygmomanometer comprising:
the integrated circuit as claimed in any one of claims 1 to 11,
wherein the sphygmomanometer further comprises, as the external sensor,
a pressure sensor which detects pressure of an arm cuff unit for inserting a subject's upper arm, and
a microphone unit which detects a sound of blood-flow in the upper arm.

13. A blood glucose meter comprising:
the integrated circuit as claimed in any one of claims 1 to 11;
an optical sensor, as the external sensor, for measuring optically a degree of coloration of examination part which is colored in accordance with the amount of glucose in blood.

14. A syringe pump comprising:
the integrated circuit as claimed in any one of claims 8 to 11;
the functional complement integrated circuit which is connected to the integrated circuit and complements a part of functions of the arithmetic control unit; and
an occlusion pressure sensor, as the external sensor, which monitors the occlusion state of a syringe.

15. A infusion pump comprising:
the integrated circuit as claimed in any one of claims 8 to 11;
the functional complement integrated circuit which is connected to the integrated circuit and complements the part of functions of the arithmetic control unit; and
an occlusion pressure sensor, as the external sensor, which monitors the occlusion state of an infusion tube.
